# EUROPEAN PATENT SPECIFICATION

(11) **EP 2 736 471 B1**
(45) Date of publication and mention of the grant of the patent: **13.01.2016**
(21) Application number: 12735919.8
(22) Date of filing: 20.07.2012
(51) Int. Cl.: A61H 19/00, A61H 23/02, A61F 13/20, A61K 9/00, A61F 13/34

(54) **TAMPON HAVING A HOLLOW SPACE**
TAMPON MIT EINEM HOHLRAUM
TAMPON AYANT UN ESPACE CREUX

(30) Priority: 28.07.2011 EP 11175780
(43) Date of publication of application: 04.06.2014
(73) Proprietor: Marlafin AG, 6332 Hagendorn (CH)
(72) Inventor: LOPEZ, Odilo, CH-8224 Löhningen (CH); HAMMEN, Axel, 5426 Lengnau (CH)
(74) Representative: AMMANN PATENTANWÄLTE AG BERN
(86) International application number: PCT/EP2012/064332
(87) International publication number: WO 2013/014098

(56) References cited:
- EP-A1- 0 415 087
- US-A- 6 036 666
- US-A1- 2007 260 210

## Description

The invention relates to a tampon of an absorbent material that has a hollow space for receiving a capsule.

From references US5782779, US6183428, and US2007/0260210, tampon assemblies having a vibration element embedded in the tampon body are known in the art. Tampon assemblies of this kind are used to relieve menstrual problems. As to the embedding of the vibration element in the tampon body, it follows from US2007/0260210 only that the vibration element is contained in a capsule on the exterior of which retaining elements are arranged in order to prevent that the capsule is pulled out of the tampon body particularly when the assembly is retracted from the vagina. In this reference, the tampon body is described as an absorbent body having a hollow space into which the capsule is apparently inserted. However, neither the construction of this tampon body nor its manufacture are described in the cited references.

On the background of this prior art, the invention is based upon the object of suggesting a tampon having a hollow space for receiving a capsule that is simple and inexpensive to manufacture. In the context of the present invention, the term "capsule" shall not be limited to a vibration element.

According to the invention, this object is attained in that the hollow space is formed by a hollow body that is surrounded by absorbent material on at least part of its length.

In particular, this solution offers the advantage that the hollow body can be prefabricated and covered, e.g. wrapped, with the absorbent material. The absorbent material may e.g. be a fibrous material, but in the present context, the term is meant to include any material that is capable of and suitable for absorbing liquids, e.g. also a material having a foamy structure.

According to one embodiment, the hollow body has retaining means for a capsule to be received in its interior. This allows a safe anchorage of this capsule in the tampon.

According to a further embodiment, the retaining means are designed as a circumferential groove arranged in the interior of the hollow body. Such a groove is simple to manufacture and is capable of receiving at least one projection provided on the circumference of the capsule.

According to other embodiments, the retaining means are designed as at least one detent and the detent is arranged at the free end of a flexible tongue. The detent may engage the free end of a capsule or engage in a recess provided on the circumference of the capsule, e.g. an annular groove, and thus retain the capsule inside the tampon.

According to a further embodiment, the hollow body is provided with at least one anchoring element that is embedded in the absorbent material. In this manner it is reliably prevented that the hollow body may be pulled out of the absorbent material.

According to a further embodiment, the anchoring element has an elongated shape, is oriented at least approximately parallelly to the longitudinal axis of the hollow body, and is connected thereto in at least one end region of the hollow body. This design allows a strip of an absorbent material to be retained between the circumference of the hollow body and the anchoring element and subsequently to wrap the absorbent material around the hollow body and the anchoring element in order to form a tampon.

According to another embodiment the hollow body has an apertured circumferential surface. This design in the manner of a cage allows incorporating more absorbent material than e.g. in a sleeve of the same outside dimensions having closed walls.

According to a particular embodiment, the hollow body is provided with an antimicrobial substance at least on its inner surface. In particular, infections are thus prevented if a capsule used in the tampon has been insufficiently cleaned. More specifically, the antimicrobial substance may e.g. be or contain silver and may be applied to the hollow body as a coating or incorporated in the material of the hollow body in the form of particles, e.g. nanoparticles.

A further aspect of the invention relates to a device for removing a capsule contained in a tampon. This device comprises an element having an opening whose clear diameter at most corresponds to the clear diameter of the hollow body.

These characteristic features offer the advantage that a capsule received in the tampon can be pulled out through the opening while the tampon is being retained by the element.

According to one embodiment of the device, the element has an open radial slot that extends from its opening. If a capsule provided with a pulling element is being used, e.g. a withdrawal string or a cable, this offers the advantage that the pulling element can be placed in this slot. According to a further embodiment, the element has releasing means for displacing retaining means in the form of detents. This allows an easy and reliable removal of a capsule contained in the hollow space of the tampon. These releasing means are preferably designed as a conical sleeve. The latter pushes the detents outwards when the tampon is moved toward the conical sleeve.

According to a further embodiment, the device has a receptacle for receiving the tampon. In this manner the tampon need not be touched in order to remove the capsule and may be disposed of together with the receptacle.

According to a further embodiment, the receptacle consists of a sleeve that is closed on one side. Such a receptacle is particularly simple to manufacture.

Ultimately, according to another embodiment, the sleeve is divided in the longitudinal direction and composed of two half-shells that are preferably connected by a hinge. Such a receptacle is particularly easy to handle.

Exemplary embodiments of the invention will be explained by way of examples in more detail hereinafter with reference to the accompanying drawings showing
- Figure 1: a partly cutaway perspective view of a tampon,
- Figure 2: a perspective view of a hollow body,
- Figure 3: a longitudinal section of a detail of another embodiment of a tampon having a capsule with a withdrawal element inserted in the hollow body,
- Figure 4: a perspective view of a device for removing the capsule, and
- Figures 5a and 5b: a sectional view of another device for removing the capsule.

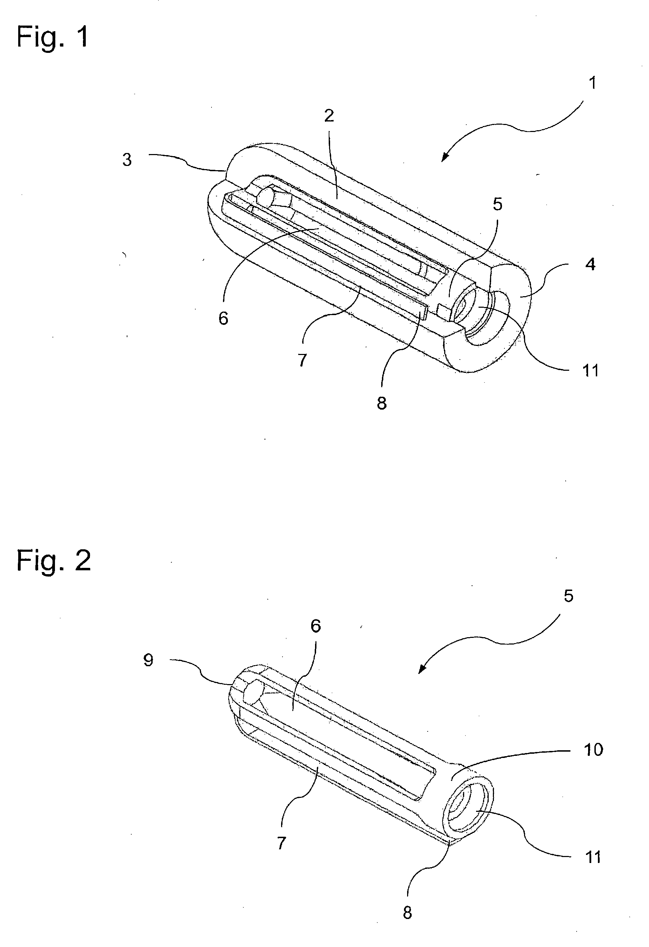

Figure 1 shows a tampon 1 in a perspective view. It consists of an absorbent material 2 and has a rounded front end 3 and a rear end 4. Tampon 1 need not be cylindrical as depicted but may alternatively have a different longitudinal contour and a different cross-sectional shape. In the interior of absorbent material 2 a hollow body 5 is visible that is fastened in absorbent material 2 by means of an anchoring element 7. The function of anchoring element 7 will be explained in more detail hereinafter with reference to Figure 2.

Figure 2 shows hollow body 5 of Figure 1 without the surrounding absorbent material 2. The wall of hollow body 5 is provided with apertures 6 and may therefore also be described as a cage, and has a front end 9 and a rear end 10. The hollow body preferably consists of a synthetic material and is injection-molded. In the example according to Figure 2, the rear end 10 of hollow body 5 is ring-shaped and its inner surface is provided with an annular groove 11 that serves as a retaining means for a capsule inserted in hollow body 5. The capsule may e.g. be a vibration element in the form of a capsule that is fastened in hollow body 5 by the retaining means. To this end, the capsule may be provided on its circumference with individual projections or a circumferential ridge that engages in annular groove 11. An anchoring element 7 is formed at the front end 9 of hollow body 5 which has a free end 8 and extends approximately parallelly to the longitudinal axis of hollow body 5. In the manufacture of tampon 1, a strip of an absorbent material is introduced between anchoring element 7 and the circumferential surface of hollow body 5, similarly as in a pencil clip. Subsequently the absorbent material is wound around hollow body 5 and ultimately also covers anchoring element 7. In a variant, anchoring element 7 may alternatively be designed in the manner of an eyelet, i.e. without free end 8. The completely wound up tampon may additionally be compacted as it is usual in tampon manufacture. To avoid that hollow body 5 is damaged in the process, the latter may receive a fitting mandrel during the compacting operation. If a hollow body 5 that is apertured in the manner of a cage is used, a little more absorbent material may be incorporated due to the apertures than in a closed sleeve of the same external diameter. Furthermore, if a vibrator is used, the vibrations emanating therefrom are directly transmitted to the absorbent material through the apertures.

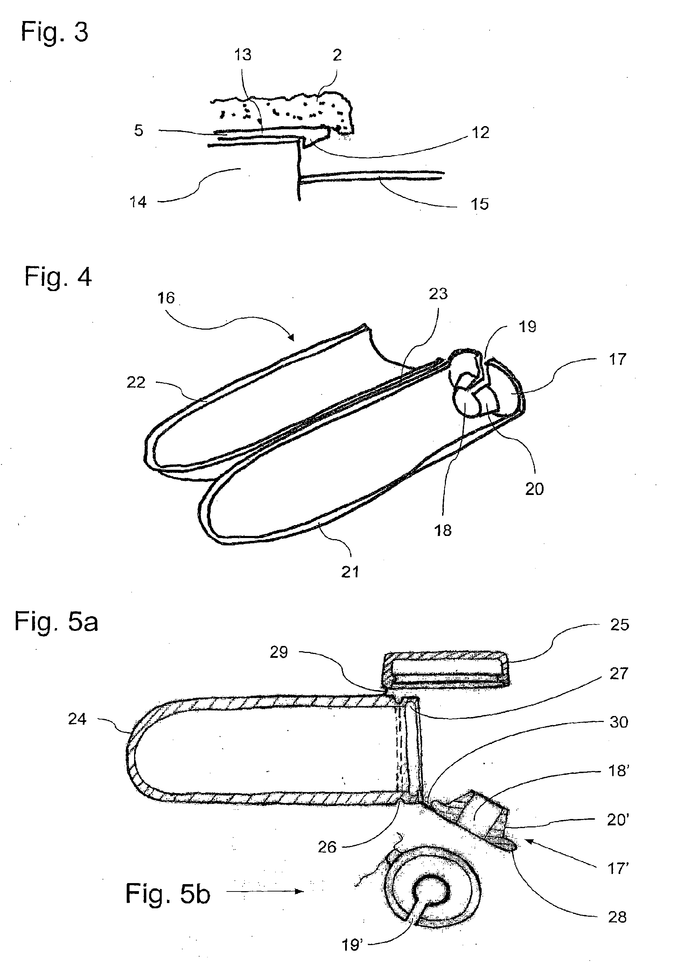

Figure 3 shows an alternative embodiment of retaining means which are designed here as at least one detent 12 that is itself arranged at the free end of a flexible tongue 13. Reference numeral 14 denotes a capsule that is being retained inside hollow body 5 by detent 12 that engages its rear end wall. Capsule 14 has a withdrawal element 15 fastened thereto which serves for withdrawing a unit consisting of tampon 1 and capsule 14 e.g. from a body orifice.

Figure 4 shows a device 16 for removing a capsule 14 from a tampon 1 of the kind described above. A disk-like element 17 has a central opening 18 through which capsule 14 fits. Furthermore, element 17 is provided with a radial slot 19 that is intended and dimensioned to receive the mentioned withdrawal element 15. In a tampon 1 having a hollow body according to Figure 2, capsule 14 is removed by inserting withdrawal element 15 in slot 19, retaining element 17, and then pulling on withdrawal element 15. To this end, the combination of annular groove 11 and of the mentioned projections or of the ridge, respectively, must be so designed that the force required for withdrawing capsule 14 from tampon 1 is significantly greater than the force required for withdrawing the tampon from a body orifice. In an advantageous further development, element 17 has a cone 20 that is intended to displace detent 12 according to Figure 3 so as to release capsule 14 when the tampon is being moved toward cone 20 by pulling on withdrawal element 15.

To ensure that tampon 1 need not be touched by hand when removing capsule 14, the device is preferably provided with a receptacle. In the example according to Figure 4, the receptacle is composed of two half-shells 21, 22 that are connected by a film hinge 23, and element 17 is fastened to one 21 of the half-shells. To remove capsule 14, tampon 1 is retained by the withdrawal element and placed in half-shell 21, and withdrawal element 15 is inserted in slot 19. Thereafter, a pull is exerted on withdrawal element 15 so that detents 12 enter into contact with cone 20 and as a result are forced outwards, thereby releasing capsule 14. The latter can now be withdrawn through opening 18. Before or after pulling on withdrawal element 15, the half-shells can be closed and after removing capsule 14, the used tampon 1 can be disposed of together with device 16.

Figures 5a and 5b show an alternative embodiment of the device where the receptacle is designed as a sleeve 24 that is closed on one side. Element 17' is connected to sleeve 24 by means of a film hinge 30 so as to be insertable, after the introduction of a used tampon into the sleeve, in the open end of the latter where it is retained by the engagement of edge 28 in an annular groove 27. This device is additionally provided with a lid 25 that may be connected to sleeve 24 by a film hinge 29 and may be pushed over the open end of sleeve 24 after the removal of capsule 14, where it engages in an outer annular groove 26. In this manner, the receptacle is tightly closed for its disposal. A corresponding lid may also be provided in the example according to Figure 4. In a top view of element 17', Figure 5b shows that the latter is also provided with a slot 19' for receiving withdrawal element 15. Half-shells 21, 22 or sleeve 24 may e.g. consist of a synthetic material or of cardboard.

From the preceding description, the one skilled in the art may derive variations without leaving the scope of the invention which is defined by the claims.

### List of Reference Numerals

- 1: tampon
- 2: absorbent material
- 3: front end
- 4: rear end
- 5: hollow body
- 6: apertures
- 7: anchoring element
- 8: free end of 7
- 9: front end
- 10: rear end
- 11: annular groove
- 12: detent
- 13: flexible tongue
- 14: capsule
- 15: withdrawal element
- 16: device
- 17, 17': element
- 18, 18': opening
- 19, 19': slot
- 20, 20': cone
- 21: half-shell
- 22: half-shell
- 23: film hinge
- 24: sleeve
- 25: lid
- 26: annular groove (exterior)
- 27: annular groove (interior)
- 28: edge
- 29: film hinge
- 30: film hinge

## Claims

1. Tampon (1) of an absorbent material (2) having a hollow space for receiving a capsule (14), **characterized in that** the hollow space is formed by a hollow body (5) that is open on at least one side and is surrounded by the absorbent material (2) on at least part of its length.

2. Tampon according to claim 1, **characterized in that** the hollow body (5) has retaining means (11, 12) for a capsule (14) to be received in its interior.

3. Tampon according to claim 2, **characterized in that** the retaining means are designed as a circumferential groove (11) arranged in the interior of the hollow body (5).

4. Tampon according to claim 2, **characterized in that** the retaining means are designed as at least one detent (12).

5. Tampon according to claim 4, **characterized in that** the detent (12) is arranged at the free end of a flexible tongue (13).

6. Tampon according to any one of the preceding claims, **characterized in that** the hollow body (5) has at least one anchoring element (7) that is embedded in the absorbent material.

7. Tampon according to claim 6, **characterized in that** the anchoring element (7) has an elongated shape, is oriented at least approximately parallelly to the longitudinal axis of the hollow body (5), and is connected thereto in at least one end region (9) of the hollow body (5).

8. Tampon according to any one of the preceding claims, **characterized in that** the hollow body (5) has an apertured circumferential surface.

9. Tampon according to any one of the preceding claims, **characterized in that** the hollow body (5) is provided with an antimicrobial substance at least on its inner surface.

10. Device (16) for removing a capsule (14) contained in a tampon (1) according to claim 2, **characterized in that** the device (16) comprises an element (17) having an opening (18) whose clear diameter at most corresponds to the clear diameter of the hollow body (5).

11. Device according to claim 10, **characterized in that** the element (17) has an open radial slot (19) that extends from its opening (18).

12. Device according to one of claims 10 to 11 for removing a capsule contained in a tampon (1) according to one of claims 4 to 5, **characterized in that** the element (17) has releasing means (20) for displacing the detents (12).

13. Device according to claim 12, **characterized in that** the releasing means are designed as a conical sleeve (20).

14. Device according to any one of claims 10 to 13, **characterized in that** it comprises a receptacle (21, 22; 24) for receiving the tampon (1).

15. Device according to claim 14, **characterized in that** the receptacle consists of a sleeve that is closed on one side.

16. Device according to claim 15, **characterized in that** the sleeve is divided in the longitudinal direction and composed of two half-shells (21, 22) that are preferably connected by a hinge (23).

## Patentansprüche

1. Tampon (1) aus saugfähigem Material (2) mit einem Hohlraum zur Aufnahme einer Kapsel (14), **dadurch gekennzeichnet, dass** der Hohlraum durch einen auf mindestens einer Seite offenen Hohlkörper (5) gebildet ist, der auf mindestens einem Teil seiner Länge vom saugfähigen Material (2) umgeben ist.

2. Tampon nach Anspruch 1, **dadurch gekennzeichnet, dass** der Hohlkörper (5) Rückhaltemittel (11, 12) für eine in seinem Inneren aufzunehmende Kapsel (14) hat.

3. Tampon nach Anspruch 2, **dadurch gekennzeichnet, dass** die Rückhaltemittel als im Inneren des Hohlkörpers (5) angeordnete, umlaufende Nut (11) ausgebildet sind.

4. Tampon nach Anspruch 2, **dadurch gekennzeichnet, dass** die Rückhaltemittel als mindestens eine Rastnase (12) ausgebildet sind.

5. Tampon nach Anspruch 4, **dadurch gekennzeichnet, dass** die Rastnase (12) am freien Ende einer Federzunge (13) angeordnet ist.

6. Tampon nach einem der vorangehenden Ansprüche, **dadurch gekennzeichnet, dass** der Hohlkörper (5) mindestens ein im saugfähigen Material eingebettetes Verankerungselement (7) aufweist.

7. Tampon nach Anspruch 6, **dadurch gekennzeichnet, dass** das Verankerungselement (7) länglich ausgebildet ist, mindestens annähernd parallel zur Längsachse des Hohlkörpers (5) orientiert ist und in mindestens einem Endbereich (9) des Hohlkörpers (5) mit diesem verbunden ist.

8. Tampon nach einem der vorangehenden Ansprüche, **dadurch gekennzeichnet, dass** der Hohlkörper (5) eine durchbrochene Mantelfläche aufweist.

9. Tampon nach einem der vorangehenden Ansprüche, **dadurch gekennzeichnet, dass** der Hohlkörper (5) zumindest an seiner inneren Oberfläche mit einer antimikrobiellen Substanz ausgestattet ist.

10. Vorrichtung (16) zum Entfernen einer in einem Tampon (1) nach Anspruch 2 angeordneten Kapsel (14), **dadurch gekennzeichnet, dass** die Vorrichtung (16) ein Element (17) mit einer Öffnung (18) aufweist, deren lichte Weite höchstens der lichten Weite des Hohlkörpers (5) entspricht.

11. Vorrichtung nach Anspruch 10, **dadurch gekennzeichnet, dass** das Element (17) einen von der Öffnung (18) ausgehenden, radialen, offenen Schlitz (19) aufweist.

12. Vorrichtung nach einem der Ansprüche 10 bis 11 zum Entfernen einer in einem Tampon (1) nach einem der Ansprüche 4 bis 5 angeordneten Kapsel, **dadurch gekennzeichnet, dass** das Element (17) Freigabemittel (20) zum Bewegen der Rastnasen (12) aufweist.

13. Vorrichtung nach Anspruch 12, **dadurch gekennzeichnet, dass** die Freigabemittel als konische Hülse (20) ausgebildet sind.

14. Vorrichtung nach einem der Ansprüche 10 bis 13, **dadurch gekennzeichnet, dass** sie ein Behältnis (21, 22; 24) zur Aufnahme des Tampons (1) aufweist.

15. Vorrichtung nach Anspruch 14, **dadurch gekennzeichnet, dass** das Behältnis aus einer einseitig geschlossenen Hülse besteht.

16. Vorrichtung nach Anspruch 15, **dadurch gekennzeichnet, dass** die Hülse in Längsrichtung geteilt ist und aus zwei Halbschalen (21, 22) besteht, die vorzugsweise durch ein Scharnier (23) miteinander verbunden sind.

## Revendications

1. Tampon (1) en matériau (2) absorbant ayant une cavité pour recevoir une capsule (14), **caractérisé en ce que** la cavité est formée par un corps creux qui est ouvert sur au moins un côté et est entouré sur au moins une partie de sa longueur par ledit matériau absorbant.

2. Tampon selon la revendication 1, **caractérisé en ce que** le corps creux (5) comprend des moyens de retenue (11, 12) pour une capsule destinée à être reçue dans son intérieur.

3. Tampon selon la revendication 2, **caractérisé en ce que** les moyens de retenue sont réalisés sous la forme d'une rainure circonférentielle formée à l'intérieur du corps creux (5).

4. Tampon selon la revendication 2, **caractérisé en ce que** les moyens de retenue sont réalisés sous la forme d'au moins un ergot d'encliquetage (12).

5. Tampon selon la revendication 4, **caractérisé en ce que** l'ergot d'encliquetage (12) est agencé à l'extrémité libre d'une languette élastique (13).

6. Tampon selon l'une des revendications précédentes, **caractérisé en ce que** le corps creux (5) comporte au moins un élément d'ancrage (7) emboîté dans ledit matériau absorbant.

7. Tampon selon la revendication 6, **caractérisé en ce que** ledit élément d'ancrage a une forme oblongue, est orienté au moins approximativement parallèlement à l'axe longitudinal du corps creux (5) et est relié au corps creux (5) dans au moins une région d'extrémité de celui-ci.

8. Tampon selon l'une des revendications précédentes, **caractérisé en ce que** le corps creux (5) a une surface circonférentielle ajourée.

9. Tampon selon l'une des revendications précédentes, **caractérisé en ce que** le corps creux (5) est pourvu d'une substance antimicrobienne au moins sur sa surface intérieure.

10. Dispositif (16) pour enlever une capsule (14) contenue dans un tampon (1) selon la revendication 2, **caractérisé en ce que** le dispositif (16) comprend un élément (17) ayant une ouverture (18) dont le diamètre intérieur correspond au diamètre intérieur du corps creux (5) au plus.

11. Dispositif selon la revendication 10, **caractérisé en ce que** l'élément (17) présente une fente (19) radiale ouverte s'étendant à partir de son ouverture (18).

12. Dispositif selon l'une des revendications 10 à 11 pour enlever une capsule contenue dans un tampon (1) selon l'une des revendications 4 à 5, **caractérisé en ce que** ledit élément (17) comprend des moyens de libération (20) pour déplacer les ergots d'encliquetage (12).

13. Dispositif selon la revendication 12, **caractérisé en ce que** les moyens de libération sont réalisés sous la forme d'une douille (20) conique.

14. Dispositif selon l'une des revendications 10 à 13, **caractérisé en ce qu'**il comporte un récipient (21, 22; 24) pour recevoir le tampon (1).

15. Dispositif selon la revendication 14, **caractérisé en ce que** le récipient est constitué d'une douille fermée d'un côté.

16. Dispositif selon la revendication 15, **caractérisé en ce que** la douille est divisée en direction longitudinale et composée de deux demi-coques (21, 22) qui sont préférablement reliées par une charnière (23).
